Europäisches Patentamt

European Patent Office

Office européen des brevets

(11) Publication number: **0 159 020 B1**

## EUROPEAN PATENT SPECIFICATION

(45) Date of publication of patent specification: **18.09.91**

(51) Int. Cl.5: **C07C 29/70**, C07C 37/66, C08G 79/02

(21) Application number: **85104615.1**

(22) Date of filing: **17.04.85**

(54) Polyphosphazene process.

(30) Priority: **18.04.84 US 601506**
**18.04.84 US 601505**

(43) Date of publication of application:
**23.10.85 Bulletin 85/43**

(45) Publication of the grant of the patent:
**18.09.91 Bulletin 91/38**

(84) Designated Contracting States:
**DE FR GB**

(56) References cited:
**DE-A- 2 612 642**
**FR-A- 2 130 701**
**US-A- 3 515 688**
**US-A- 3 702 833**
**US-A- 3 948 820**

(73) Proprietor: **ETHYL CORPORATION**
**Ethyl Tower 451 Florida Boulevard**
**Baton Rouge Louisiana 70801(US)**

(72) Inventor: **Sulzer, Gerald Michael**
**635 Woodstone Drive**
**Baton Rouge, La. 70808(US)**
Inventor: **Wilson, Riley Woodrow, Jr.**
**11903 N. Oakhills Parkway**
**Baton Rouge, La. 70810(US)**
Inventor: **Thomas, Dustin Harriman**
**5321 Highland Ridge Dr.**
**Baton Rouge La. 70816(US)**

(74) Representative: **Schwabe - Sandmair - Marx**
**Stuntzstrasse 16**
**W-8000 München 80(DE)**

## Description

Haloalkoxide-substituted phosphazene polymers are useful in many applications because of their flame resistance, low temperature flex properties and high temperature stability. Of these, most interest has been in the fluoroalkoxide-substituted polyphosphazene. Such compositions are disclosed in U.S. 3,515,688 and U.S. 3,700,629. Other fluoroalkoxides-substituted phosphazene polymers are disclosed in U.S. 3,702,833, U.S. 3,732,175, U.S. 3,838,073, U.S. 3,844,983, U.S. 3,888,799, U.S. 3,888,800, U.S. 3,896,058, U.S. 3,943,088, U.S. 3,945,966, U.S. 3,948,820, U.S. 3,970,533, U.S. 3,972,841, U.S. 4,000,166, which references should be consulted for their disclosure of the prior methods of making alkaline metal fluoroalkoxides and the use of such fluoroalkoxides in preparing polyphosphazene polymers and the utility of such polyphosphazene polymers.

In U.S. 3,515,688, the sodium fluoroalkoxide used to make the fluoroalkoxide-substituted phosphazene polymers was prepared by reacting in tetrahydrofuran metallic sodium directly with the fluorine-substituted alcohol. Pieces of metallic sodium, which were cut and weighed under dried benzene, were added directly to a mixture of tetrahydrofuran and the fluorine-substituted alcohol and the mixture was allowed to stand undisturbed overnight, following which it was refluxed to complete the reaction. A hazard associated with a process carried out in this manner is that the metallic sodium in addition to reacting with the alcohol hydroxyl groups can also react with the halogen bonded to the alcohol. Such reactions can become very exothermic and lead to eruption of the reaction contents from the reaction vessel and can ignite. Attempts to prepare sodium alkoxides of fluorine-substituted alcohols using sodium dispersed in tetrahydrofuran (THF) were not successful because the sodium dispersion initially made in THF tended to coalesce.

It has now been discovered that sodium dispersions made in a cycloalkane such as cyclohexane or cyclohexane containing a phenol retain their dispersed state and when added in a controlled manner to a fluorine-substituted alcohol react rapidly with the alcoholic hydroxyl group without accummulating large amounts of unreacted sodium which could lead to the hazard referred to above. The sodium fluoroalkoxide or sodium fluoroalkoxide-phenoxide mixture prepared in this manner reacts very efficiently with phosphonitrilic chloride polymers thereby replacing the chlorine atoms with fluoroalkoxide or with fluoroalkoxide and phenoxide groups.

A preferred embodiment of the invention is a process for making a sodium alkoxide of a halogen-substituted alcohol without excessive reaction of sodium with the halogen substituent, said process comprising

(a) dispersing molten metallic sodium in a cycloalkane hydrocarbon containing 5-8 carbon atoms at a pressure high enough to maintain said cycloalkane hydrocarbon in the liquid phase above the melting temperature of sodium;

(b) cooling the resultant dispersion to a temperature below the melting point of sodium; and

(c) adding preferably 0.9 - 1.0 equivalents of the resultant sodium dispersion to a solution of 1 equivalent of a halogen-substituted alcohol in an ether solvent at a temperature of -30°C up to reflux, said equivalents being based on the hydroxyl content of said halogen-substituted alcohol.

Another preferred embodiment of the invention is a process for making a mixture of a sodium alkoxide of a halogen-substituted alcohol and a sodium aryloxide of a phenol without excessive reaction of sodium with the halogen substituent, said process comprising

(a) dispersing about one equivalent of molten metallic sodium in a cycloalkane hydrocarbon containing 5-8 carbon atoms and 0.05-0.5 moles of a phenol at a pressure high enough to maintain said cycloalkane hydrocarbon in the liquid phase above the melting temperature of sodium;

(b) cooling the resultant dispersion to a temperature below the melting point of sodium; and

(c) adding the resultant dispersion to a solution of of a halogen-substituted alcohol in an ether solvent at a temperature of -30°C up to reflux the amount of halogen-substituted alcohol being sufficient to react with at least a major amount of the metallic sodium in the resultant dispersion.

Preferred cycloalkanes useful in the process are those containing 5-8 carbon atoms such as cyclopentane, cyclohexane, cycloheptane and cyclooctane. The cycloalkane ring may be substituted with alkyl groups such as methyl, ethyl, propyl and the like. The preferred cycloalkane hydrocarbon is cyclohexane.

A broad range of phenols can be used to make the dispersion. Examples include phenol, o-cresol, p-cresol, m-ethylphenol, p-ethylphenol, p-isopropylphenol, p-sec-butyl-phenol, p-tert-octylphenol, p-n-dodecylphenol, p-sec-eicosyl-phenol, α-naphthol, β-naphthol, p-methoxyphenol, m-ethoxyphenol, and the like.

The more preferred phenols are the allylsubstituted phenols. These phenols introduce allylphenoxide groups into the sodium dispersion. When the resultant sodium dispersion is used to make haloalkoxides, the mixture will also contain allylphenoxides. When this mixture is subsequently used to substitute a phosphonitrilic chloride polymer, it will introduce both haloalkoxide and allylphenoxide groups. The allylphenoxide groups are beneficial in that they impart curing properties to the resultant polyphosphazene.

Allylphenols are aromatic hydroxy compounds having an allyl group bonded to a benzene ring. Although mononuclear aromatics are preferred, the invention includes the use of allylsubstituted polynuclear phenols. Examples of suitable allylphenols include o-allyl-p-cresol, 2-allyl-4-ethylphenol, 4-allyl-phenol, 2-methyl-4-allylphenol, 2-allyl-4-chlorophenol, 2-allyl-4-fluorophenol, 2-allyl-4,6-difluorophenol, 2-allyl-4-isopropyl-phenol, 2-allyl-1-naphthol, 4-allyl-1-naphthol, 4-allyl-2-naphthol, 2-allyl-4-methoxyphenol, 2-allyl-4-ethoxyphenol, 2,4-diallylphenol, 2-allyl-4-nitrophenol, and the like. The most preferred allylphenol is ortho-allylphenol.

The amount of phenol in the cycloalkane need not be great. A range of 0.01-0.2 gram moles per each gram atom or equivalent of sodium is a useful range. In most cases, an excellent dispersion is obtained using 0.05-0.2 moles of phenol per equivalent of sodium. The phenol can be added to the cycloalkane prior to adding the sodium metal. Alternatively, it can be added after adding the sodium metal. Likewise, it can be added after heat-up to melt the sodium or even after the molten sodium is agitated to form the dispersion.

Alkali metals that can be used in the process include both sodium and potassium. Of these, sodium is most preferred because of its ready availability and low cost. The amount of sodium in the dispersion can vary over a wide range. A preferred concentration is from 20-50 weight percent sodium in the resultant sodium dispersion. A more preferred sodium concentration is from 30 to 40 weight percent.

The dispersion is prepared by adding metallic sodium to the cycloalkane hydrocarbon or cycloalkane containing a phenol and heating the mixture to a temperature above the melting point of sodium and maintaining at this temperature until the sodium becomes molten. Sodium melts at about 97.5°C which is above the normal boiling point of cyclopentane and cyclohexane. When the temperature used to make the dispersion is above the normal boiling point of the cycloalkane dispersion medium pressure equipment must be used to prepare the dispersion. Only modest pressures are encountered. For example, using cyclohexane at 105°C resulted in a pressure of 14 psig (1.98 bars absolute).

Optionally, the sodium can be pre-melted and added to the hot cycloalkane or cycloalkane containing a phenol in order to reduce processing time.

The sodium used in the process should be cleaned of oxides and hydroxides which frequently form on the surface of metallic sodium. The dispersing temperature is preferably 105-120°C although temperatures as low as 100°C can be used. The dispersion is then formed by vigorously agitating the mixture using a high shear dispersing agitator such as a conventional turbine type dispersing head. Such dispersing heads are used at very high RPM such that the tip speed of the agitator is in the vicinity of 45-90 feet per second.

Dispersing agents can be used in making the dispersions although they are generally not necessary. Useful dispersing agents include fatty acids such as oleic acid which forms sodium oleate in the dispersion. Preferably the use of a dispersing agent should be avoided because it tends to introduce an impurity into the reaction system when the alkali metal haloalkoxide is reacted with the phosphonitrilic chloride polymer. Impurities are generally detrimental to the properties of the substituted polymer and elaborate purification methods become necessary to eliminate impurities.

Halogen-substituted alcohols which can be reacted to form sodium alkoxides according to the present process include any halogen-substituted alcohol and can contain up to 20 or more carbon atoms and 41 or more halogen atoms. Halogen substituents include chlorine, fluorine, bromine and iodine. Examples of such halogen-substituted alcohols are trifluoromethanol, trichloromethanol, 2-chloroethanol, 2,2-dichloroethanol, 2,2-difluoroethanol, 2,2-dibromoethanol, 2,2,2-trifluoroethanol, 2,2,2-trichloroethanol, 2,3-dibromopropanol, 2,3-diiodopropanol, 2,2,3,3,3-pentafluoropropanol, 6,6-dichlorohexanol, 7,8-dibromooctadecanol, 7,8-difluorooctadecanol, 8,9-diiodoeicosanol and the like.

In a more preferred embodiment, the halogen-substituted alcohol is a fluorine-substituted alcohol. In a still more preferred embodiment, the fluorine-substituted alcohol is a mixture of (a) trifluoroethanol and (b) a telomer alcohol having the general formula $X(CF_2)_pCH_2OH$ wherein X is hydrogen or fluorine and p is an even integer from 2 to about 12 and mixtures of such telomer alcohols.

Preferably the mixture contains (a) 30-70 wt% trifluoroethanol and (b) 70-30 wt% of the telomer alcohol. In the most preferred embodiment, the fluorine-substituted alcohol is a mixture of (a) 30-70 wt% trifluoroethanol and (b) 70-30 wt% of a mixture of telomer alcohols. Most preferably X is hydrogen.

The alcohol reactant can include halogen-free alcohols as well as phenols when the product is desired to contain alkoxides or aryloxides of such alcohols and/or phenols. However, in order for the process to be beneficial the alcohol reactant should contain a substantial amount of halogen-substituted alcohol. For example, alcohol mixtures containing 10 wt% or more halogen-substituted alcohols can benefit from the present process.

The haloalcohol is dissolved in an ether solvent. A wide range of ethers can be used provided they are substantially inert under the reaction conditions. It is highly preferred that the ether solvent also be capable of dissolving the haloalkoxide- or haloalkoxide and phenoxide-substituted polyphosphazene formed when the sodium haloalkoxide or mixture of sodium haloalkoxide and phenoxide is reacted with phosphonitrilic chloride polymer. Useful solvents are diethyl ether, ethyl butyl ether, dibutyl ether, dioxane, dimethoxy ethane, diethoxy ethane, dimethyl ether of diethylene glycol, dibutoxy ethane, dibutyl ether of diethylene glycol. Most work has been carried out using tetrahydrofuran (THF) as the haloalcohol solvent.

The amount of ether solvent should be an amount which will dissolve at least a major portion of the sodium haloalkoxide formed in the reaction. More preferably the amount is sufficient to dissolve substantially all of the sodium haloalkoxide formed in the reaction. The amount required will vary with what ether is selected and what haloalkoxide is prepared. The required amount can be determined experimentally with very little effort. A useful range in which to test is 300-5000 parts by weight ether for each 100 parts of haloalcohol. A more preferred range is 350-1000 parts of ether solvent per 100 parts haloalcohol. When making a sodium fluoroalkoxide using the preferred telomer alcohols good results have been achieved with 300-400 parts THF per 100 parts telomer alcohol.

The sodium dispersion is added to the halogen-substituted alcohol at a rate such that there is not a large amount of unreacted sodium in the halogen-substituted alcohol at any time. The rate of sodium reacting with the alcohol hydroxyl to form alkoxide can be followed by measuring hydrogen evolution. Generally, good results have been obtained by limiting sodium feed such that there is less than 0.02 wt% unreacted sodium in the reaction mixture at any time.

Alternatively, the sodium dispersion containing allylphenoxides is added to the halogen-substituted alcohol at a rate such that there is not a large amount of unreacted sodium in the halogen-substituted alcohol at any time. The rate of sodium reacting with the alcohol hydroxyl to form alkoxide can be followed by measuring hydrogen evolution. Generally, good results have been obtained by limiting sodium feed such that there is less than 0.02 wt% unreacted sodium in the reaction mixture at any time.

The temperature of the reaction mixture need not be raised. Preferably the reaction temperature is maintained at -20 to +30°C. Excellent results with no noticeable halogen reaction have been obtained at -10 to +20°C. The preferred reaction temperature is -5 up to 10°C.

The amount of sodium dispersion added to the halogen-substituted alcohol should be an amount which is sufficient to react with most of the alcohol hydroxyl groups. If an excess of sodium dispersion is added to the halogen-substituted alcohol, the amount of alcohol should be sufficient to consume most of the sodium. Preferably, the amount of alcohol should consume at least 90 wt% of the sodium metal in the dispersion. A preferred amount is 0.8-1.5 equivalent of sodium per each equivalent of alcohol hydroxyl. A more preferred range is 0.9-1.0 equivalents of sodium per alcohol hydroxyl equivalent. Most preferably about one equivalent of sodium is added for each equivalent of alcohol hydroxyl.

Addition time for the sodium dispersion is generally ten minutes up to eight hours. The reaction mixture is stirred during this time under an inert atmosphere such as nitrogen. After completion of the addition, the mixture is stirred for a short period up to about two hours. Any unreacted sodium remaining at this time can be removed by filtration. The following examples show how the process is conducted.

Example 1

In a pressure resistant dispersion vessel fitted with a high speed turbine type dispersing head was placed 300 parts of cyclohexane. The vessel was purged with nitrogen and 200 parts of clean sodium pieces were dropped into the cyclohexane. The dispersion vessel was sealed and heated to 105°C at which time pressure had increased to about 14 psig (1.98 bars absolute). The high speed agitator was started and run at about 14,000-16,000 rpm for 13 minutes to disperse the molten sodium. At this time, the agitator was stopped and the mixture permitted to slowly cool to a temperature below the melting point of sodium resulting in a stable sodium dispersion in cyclohexane.

Example 2

In the dispersing vessel of Example 1 was placed 255 grams of cyclohexane and 170 grams of sodium pieces. The vessel was purged with nitrogen and sealed and heated to 110°C. The high shear agitator was started and run at 9,600 rpms. The dispersion was not completely stable so agitator speed was increased to 11,500 rpm and run at this speed for 20 minutes. Agitation was stopped and the dispersion cooled to about 60°C. Particle size was too large for easy discharge through a one-quarter inch tubing so the mixture was again heated to about 110°C. The high shear agitator was started and run at 13,300 rpms for 20 minutes. The agitator was stopped and the dispersion allowed to cool to 40°C. The particle size was very small and the appearance of the dispersion was good, and the material easily transferred through a clean 1/4" tube.

Example 3

In a reaction vessel fitted with stirrer and thermometer was placed 1490 g tetrahydrofuran, 182g 2,2,2-trifluoroethanol and 191g of telomer alcohol in which X is fluorine, p is 2-8 and has an average value of about 4. This was stirred under nitrogen and over a period of 3 hours. 60g of the sodium dispersion made in Example 1 was added while maintaining the temperature in the range of -30°C to 4°C. The reaction went smoothly giving a solids-free sodium fluroalkoxide product.

This example was repeated using 110 minute sodium dispersion feed time with excellent results.

Example 4

In a pressure resistant dispersion vessel equipped with a 3/4" diameter turbine-type high shear agitator was placed 273g of cyclohexane and 170g of clean sodium. The vessel was flushed with nitrogen and sealed. The vessels contents were heated to about 105°C to melt the sodium. When the sodium was melted, the agitator was started and 0.7 ml of o-allylphenol was injected into the vessel. The agitator was run thirty minutes at 14,000 rpms and then stopped. The vessel was allowed to cool to 64° at which time it was discharged resulting in a very fine stable sodium dispersion.

In a second reactor was placed 1,490g of tetrahydrofuran, 182g of tri-fluoroethanol and 190g of telomer fluoroalcohols. While stirring under nitrogen at about 0-5°C a quantity of the above sodium dispersion which contained about 61g of sodium metal was fed over 2.6 hours. The mixture was then stirred at 15-20°C for 40 minutes to complete the reaction. Less than 1g of sodium remained unreacted.

Example 5

Another dispersion was made similar to Example 4 using 170g sodium, 274g cyclohexane and 0.86g o-allylphenol. A quantity of this dispersion containing about 60g of sodium metal was fed under nitrogen to a third solution of 180g of trifluoroethanol, 180g of telomer fluoroalcohols and 1600g of tetrahydrofuran at 0-5°C over a 120 minute period. No sodium metal was detected following the reaction.

The alkali metal haloalkoxides and mixtures of haloalkoxides and allylphenoxides made by the process are used to insert haloalkoxide and allylphenoxide groups on phosphonitrilic chloride polymers (chloropolymers). Chloropolymers are well known compositions and come in a wide range of molecular weights. At one extreme are the low molecular weight cyclic chloropolymers containing 3-7 $(PNCl_2)$ units such as phosphonitrilic chloride trimer and tetramer made by the reaction of $PCl_5$ and $NH_4Cl$ in approximately equal mole amounts in a solvent such as monochlorobenzene. Slightly higher molecular weight oligomer products can be made by the same general reaction using a molar excess of $PCl_5$. These oligomers usually contain about 3-9 $(PNCl_2)$ units.

The cyclic polymers can be converted to high molecular weight chloropolymers by heating the purified cyclic chloropolymer to temperatures in the range of 220-275°C. The low molecular weight oligomers can be converted to high molecular weight chloropolymers by heating with $NH_4Cl$ at a temperature of 130-200°C as described in U.S. 4,374,815.

Although the sodium haloalkoxides amd mixtures of sodium haloalkoxides and allylphenoxides made by the present process can be reacted with any chloropolymer to introduce haloalkoxide group or both allylphenoxide and haloalkoxide groups, it is preferred that the chloropolymer contain at least about 10 $(PNCl_2)$ units. More preferably, the chloropolymer is a high molecular weight substantially linear chloropolymer containing over 100 $(PNCl_2)$ units and most preferably over 1,000 such units.

The haloalkoxide groups and mixtures of haloalkoxide and allylphenoxide groups are introduced into the chloropolymer by dissolving the chloropolymer in an inert solvent and then adding the ether solution of the haloalkoxide or mixture of haloalkoxide and allylphenoxide. Suitable solvents for the chloropolymer are ethers, aromatics, cycloaliphatics and ketones. Representative examples of such solvents are tetrahydrofuran, diethyl ether, dibutyl ether, dioxane, dimethoxyethane, dimethylether of diethylene glycol, benzene, toluene, xylene, cyclopentane, cylcohexane, cycloheptane, cyclooctane, cyclododecane, methylcyclohexane, acetone, methyl ethyl ketone, diethyl ketone, dibutyl ketone and the like. Preferred solvents are tetrahydrofuran, toluene and cyclohexane.

The amount of solvent should be sufficient to dissolve all or most of the chloropolymer at reaction temperature. Generally, a stable solution is obtained using 10-50 Kg of solvent per Kg of chloropolymer. The high molecular weight polymers are slow to dissolve so require stirring for several hours, preferably at slightly elevated temperatures of 40-50°C. Preferably the solvent is maintained under an inert dry nitrogen atmosphere during solvation.

The substitution reaction is conducted at moderate temperatures. A useful temperature range is 25-100°C. A more preferred temperature range is 40-70°C. The reaction is conducted until the desired degree of substitution is obtained or alternatively until the chloride content of the chloropolymer is reduced to the desired level. The reaction is usually complete in 4-12 hours.

The amount of sodium haloalkoxide or haloalkoxide phenoxide solution used should be an amount which contains sufficient sodium haloalkoxide or sodium haloalkoxide and phenoxide mixture to provide the desired degree of substitution. Generally, the solution contains 0.9-1.1 moles of sodium haloalkoxide per equivalent of replaceable chloride in the chloropolymer. Alternately, the solution contains 0.8-1.05 moles of sodium haloalkoxide and 0.05-0.2 moles of sodium phenoxide preferably allylphenoxide per equivalent of replaceble chloride in the chloropolymer.

The following examples show how the sodium haloalkoxides or sodium haloalkoxides and allylphenoxides made according to the present process are used in a substitution reaction with chloropolymer.

Preparation - Example 6

The chloropolymer was made by placing 192.5 grams of purified polymer grade phosphonitrilic chloride cyclic trimer and a catalytic amount (0.12 wt%) of a molar complex of $BCl_3$ and triphenyl phosphate in a clean dry polymerization tube. The tube was sealed and placed in an oven at 220°C for 21 hours. Chloropolymer was recovered by dissolving the tube's contents in 800 ml of cyclohexane and then adding 2 liters of heptane to precipitate the high molecular weight chloropolymers while maintaining low molecular weight and cyclic chloropolymers in solution. The liquid phase was removed and discarded and the coagulated chloropolymer was washed with additional heptane. The chloropolymer was then dissolved in cyclohexane ready for use in the substitution reaction.

Example 7

A substitution reaction was conducted by placing 749.9 grams of a sodium fluoroalkoxide solution made as in Example 3 by the addition of a dispersion of 60 grams of sodium in 90 grams of cyclohexane to a mixture of 180 grams of trifluoroethanol and 180 grams of telomer alcohol in 1450g of tetrahydrofuran. To this was added 741.8 grams of dry tetrahydrofuran and 3.7 grams of distilled orthoallylphenol. The solution was stirred under dry nitrogen and heated to 45°C. The chloropolymer solution from Preparation - Example 6 was slowly added over a 30 minute period at 45-60°C. Stirring was continued for 8 hours at 67°C.

Work up was carried out by injecting dry carbon dioxide through the mixture to neutralize the mixture to a pH of about 6.5. Then 86 grams of 10 wt% aqueous sodium bromide was added to agglomerate the NaCl. This mixture was centrifuged and the organic liquid phase was recovered and poured into 10 liters of a heptane-hexane mixture to precipitate the polyphosphazene gum. The gum was redissolved in acetone and precipitated with 8 liters of water. The precipitated gum was dried under vacuum at 60°C to give a fluoroalkoxide-substituted linear polyphosphazene.

Example 8

An aliquot of the sodium fluoroalkoxides containing allylphenoxides from Example 5 containing 1g equivalent of sodium was placed in a reaction vessel fitted with a stirrer and heating means. The vessel was purged with nitrogen. Then a 10 wt% solution containing 54g of high molecular weight chloropolymer in cyclohexane was pumped into the reaction vessel over a ten minute period. Temperature rose from 25°C to 48°C. Stirring was continued for six hours at 60°C. At completion, the pH was about 7. Two drops of sulfuric acid was added to lower pH to about 5. Then aqueous sodium chloride solution was added to remove salt. The solvents were steam distilled leaving a substituted polyphosphazene gum. This gum was dried resulting in 148g of a useful fluoroalkoxy-substituted polyphosphazene containing sufficient allylphenoxide groups to impart cure properties to the elastomer.

**Claims**

1. A process for making a sodium alkoxide of a halogen-substituted alcohol without excessive reaction of sodium with the halogen substituent, said process comprising

   (a) dispersing molten metallic sodium in a cycloalkane hydrocarbon containing 5-8 carbon atoms at a pressure high enough to maintain said cycloalkane hydrocarbon in the liquid phase above the melting temperature of sodium;

   (b) cooling the resultant dispersion to a temperature below the melting point of sodium; and

   (c) adding 0.9 - 1.0 equivalents of the resultant sodium dispersion to a solution of 1 equivalent of a halogen-substituted alcohol in an ether solvent at a temperature of -30°C up to reflux, said equivalents being based on the hydroxyl content of said halogen-substituted alcohol.

2. A process as claimed in Claim 1 in which said sodium alkoxide of a halogen-substituted alcohol is prepared as a mixture with a sodium aryloxide of an allylphenol without excessive reaction of sodium with the halogen substituent, said process comprising
(a) dispersing about one equivalent of molten metallic sodium in a cycloalkane hydrocarbon having 5-8 carbon atoms and 0.05-0.2 moles of a phenol dispersant at a pressure high enough to maintain said cycloalkane hydrocarbon in the liquid phase above the melting temperature of sodium;
(b) cooling the resultant dispersion to a temperature below the melting point of sodium; and
(c) adding said resultant sodium dispersion to a solution of a halogen-substituted alcohol in an ether solvent at a temperature of -30°C up to reflux, the amount of said halogen-substituted alcohol being sufficient to react with at least 90 wt% of the metallic sodium in said resultant sodium dispersion.

3. A process for making a haloalkoxide-substituted or haloalkoxide- and phenoxide-substituted polyphosphazene, said process comprising the steps of:
(a) dissolving a phosphonitrilic chloride polymer in an inert solvent to form a polymer solution;
(b) adding said polymer solution to an ether/cycloalkane solution of a sodium alkoxide of a halogen-substituted alcohol made by the process of Claim 1 or 2 whereby halogen-substituted alkoxide or mixture of halogen-substituted alkoxide and allylphenoxide replaces at least part of the chloride atoms bonded to phosphorus forming haloalkoxide-substituted or haloalkoxide- and allylphenoxide-substituted polyphosphazene; and
(c) recovering said haloalkoxide-substituted polyphosphazene.

4. A Process as claimed in Claims 1, 2, or 3 wherein said cycloalkane hydrocarbon is cyclohexane.

5. A process as claimed in Claims 1, 2 or 3 wherein said ether solvent is tetrahydrofuran.

6. A process as claimed in Claims 2 or 3 in which said phenol is an allylphenol.

7. A process as claimed in Claims 2 or 3 wherein said halogen-substituted alcohol is a fluorine-substituted alcohol.

8. A process as claimed in Claim 7 wherein said fluorine-substituted alcohol is a mixture of (a) trifluoroethanol and (b) a telomer alcohol which has the formula:

$$X(CF_2)_pCH_2OH$$

wherein X is selected from hydrogen and fluorine and p is an integer from 2-12 and mixtures thereof.

9. A process as claimed in Claim 8 wherein X is hydrogen.

10. A process as claimed in Claim 3 wherein said phosphonitrilic chloride polymer is a substantially linear polymer containing at least 100 $(PNCl_2)$ units.

11. A process as claimed in Claim 3 wherein said phosphonitrilic chloride polymer is a cyclic chloropolymer containing about 3-7 $(PNCl_2)$ units.

12. A process as claimed in Claim 11 wherein said cyclic chloropolymer is mainly trimer, tetramer or mixtures thereof.

**Revendications**

1. Procédé de production d'un alcoolate de sodium d'un alcool à substituant halogéno sans réaction excessive du sodium avec le substituant halogéno, ledit procédé consistant
(a) à disperser du sodium métallique fondu dans un hydrocarbure du type d'un cycloalcane contenant 5 à 8 atomes de carbone à une pression suffisamment haute pour maintenir ledit hydrocarbure du type d'un cycloalcane en phase liquide au-dessus de la température de fusion du sodium ;
(b) à refroidir la dispersion résultante à une température inférieure au point de fusion du sodium ; et
(c) à ajouter 0,9 à 1,0 équivalent de la dispersion de sodium résultante à une solution de 1 équivalent d'un alcool à substituant halogéno dans un solvant du type d'un éther à une température allant de -30°C au reflux, lesdits équivalents étant basés sur la teneur en hydroxyle dudit al-

**2.** Procédé suivant la revendication 1, dans lequel ledit alcoolate de sodium d'un alcool à substituant halogéno est préparé sous forme d'un mélange avec un arylate de sodium d'un allylphénol sans réaction excessive de sodium avec le substituant halogéno, ledit procédé consistant

(a) à disperser environ 1 équivalent de sodium métallique fondu dans un hydrocarbure du type d'un cycloalcane ayant 5 à 8 atomes de carbone et 0,05 à 0,2 mole d'un dispersant phénolique à une pression suffisamment haute pour maintenir ledit hydrocarbure du type d'un cycloalcane en phase liquide au-dessus de la température de fusion du sodium ;

(b) à refroidir la dispersion résultante à une température inférieure au point de fusion du sodium ; et

(c) à ajouter ladite dispersion de sodium résultante à une solution d'un alcool à substituant halogéno dans un solvant du type d'un éther à une température allant de -30° C au reflux, la quantité dudit alcool à substituant halogéno étant suffisante pour réagir avec au moins 90 % en poids du sodium métallique dans ladite dispersion de sodium résultante.

**3.** Procédé de production d'un polyphosphazène à substituant halogénalcoolate ou à substituant halogénalcoolate et phénate, ledit procédé comprenant les étapes consistant :

(a) à dissoudre un polymère de chlorure phosphonitrilique dans un solvant inerte pour former une solution de polymère ;

(b) à ajouter ladite solution de polymère à une solution dans un éther et un cycloalcane d'un alcoolate de sodium d'un alcool à substituant halogéno préparé par le procédé suivant la revendication 1 ou 2 de manière que l'alcoolate à substituant halogéno ou le mélange d'allylphénate et d'alcoolate à substituant halogéno remplace au moins une partie des atomes de chlore liés au phosphore en formant un polyphophazène à substituant halogénalcoolate ou à substituant halogénalcoolate et allylphénate ; et

(c) à recueillir ledit polyphosphazène à substituant halogénalcoolate.

**4.** Procédé suivant la revendication 1, 2 ou 3, dans lequel l'hydrocarbure du type d'un cycloalcane est le cyclohexane.

**5.** Procédé suivant la revendication 1, 2 ou 3, dans lequel ledit solvant du type éther est le tétrahydrofuranne.

**6.** Procédé suivant la revendication 2 ou 3, dans lequel le phénol est un allylphénol.

**7.** Procédé suivant les revendications 2 ou 3, dans lequel l'alcool à substituant halogéno est un alcool substitué par du fluor.

**8.** Procédé suivant la revendication 7, dans lequel ledit alcool substitué par du fluor est un mélange (a) de trifluoréthanol et (b) d'un alcool télomérique qui répond à la formule :

$$X(CF_2)_pCH_2OH$$

dans laquelle X est choisi entre l'hydrogène et le fluor et p est un nombre entier de 2 à 12, et leurs mélanges.

**9.** Procédé suivant la revendication 8, dans lequel X est l'hydrogène.

**10.** Procédé suivant la revendication 3, dans lequel ledit polymère de chlorure phosphonitrilique est un polymère pratiquement linéaire contenant au moins 100 motifs $(PNCl_2)$.

**11.** Procédé suivant la revendication 3, dans lequel ledit polymère de chlorure phosphonitrilique est un chloropolymère cyclique contenant environ 3 à 7 motifs $(PNCl_2)$.

**12.** Procédé suivant la revendication 11, dans lequel ledit chloropolymère cyclique est principalement le trimère, le tétramère ou leurs mélanges.

**Patentansprüche**

**1.** Verfahren zur Herstellung eines Natriumalkoxids aus einem halogensubstituierten Alkohol ohne übermäßige Reaktion von Natrium mit dem Halogensubstituenten, bei dem man:

(a) geschmolzenes metallisches Natrium in einem Cycloalkankohlenwasserstoff, der 5-8 Kohlenstoffatome enthält, bei einer Temperatur, die hoch genug ist, um den Cycloalkankohlenwasserstoff in der flüssigen Phase oberhalb der Schmelztemperatur von Natrium zu halten, dispergiert;

(b) die erhaltene Dispersion auf eine Temperatur unterhalb des Schmelzpunktes von Natrium abkühlt; und

(c) 0,9 - 1,0 Äquivalente der erhaltenen Natriumdispersion zu einer Lösung von 1 Äquivalent eines halogensubstituierten Alkohols in einem Etherlösungsmittel bei einer Temperatur von -30 °C bis zum Rückfluß zugibt, wobei sich die Äquivalente auf den Hydroxylgehalt des halogensubstituierten Alkohols beziehen.

2. Verfahren nach Anspruch 1, bei dem man das Natriumalkoxid aus einem halogensubstituierten Alkohol als eine Mischung mit einem Natriumaryloxid aus einem Allylphenol ohne übermäßige Reaktion des Natriums mit dem Halogensubstituenten herstellt, bei dem man:
(a) etwa ein Äquivalent von geschmolzenem Natrium in einem Cycloalkankohlenwasserstoff, der 5-8 Kohlenstoffatome und 0,05-0,2 Mol von einem Phenoldispergiermittel aufweist, bei einem Druck, der hoch genug ist, um den Cycloalkankohlenwasserstoff in der flüssigen Phase oberhalb der Schmelztemperatur von Natrium zu halten, dispergiert;
(b) die erhaltene Dispersion auf eine Temperatur unterhalb des Schmelzpunktes von Natrium abkühlt; und
(c) die erhaltene Natriumdispersion zu einer Lösung von einem halogensubstituierten Alkohol in einem Etherlösungsmittel bei einer Temperatur von -30 °C bis zum Rückfluß zugibt, wobei die Menge des halogensubstituierten Alkohols ausreichend ist, um mit mindestens 90 Gew.-% des metallischen Natriums in der erhaltenen Natriumdispersion zu reagieren.

3. Verfahren zur Herstellung eines haloalkoxidsubstituierten oder haloalkoxid- und phenoxidsubstituierten Polyphosphazens, bei dem man:
(a) ein Phosphonitrilchloridpolymer in einem inerten Lösungsmittel zur Bildung einer Polymerlösung auflöst;
(b) die Polymerlösung zu einer Ether/Cycloalkan-Lösung von einem Natriumalkoxid von einem halogensubstituierten Alkohol, hergestellt nach dem Verfahren gemäß Anspruch 1 oder 2, zugibt, wodurch das halogen-substituierte Alkoxid oder die Mischung von einem halogen-substituierten Alkoxid und Allylphenoxid mindestens einen Teil der Chloridatome ersetzt, die an den Phosphor gebunden sind, wobei ein haloalkoxid-substituiertes oder haloalkoxid- und allylphenoxid-substituiertes Polyphosphazen gebildet wird; und
(c) das haloalkoxid-substituierte Polyphosphazen zurückgewinnt.

4. Verfahren nach Anspruch 1, 2 oder 3, bei dem der Cycloalkankohlenwasserstoff Cyclohexan ist.

5. Verfahren nach Anspruch 1, 2 oder 3, bei dem das Etherlösungsmittel Tetrahydrofuran ist.

6. Verfahren nach Anspruch 2 oder 3, bei dem das Phenol ein Allylphenol ist.

7. Verfahren nach Anspruch 2 oder 3, bei dem der halogensubstituierte Alkohol ein fluorsubstituierter Alkohol ist.

8. Verfahren nach Anspruch 7, bei dem der fluorsubstituierte Alkohol eine Mischung von (a) einem Trifluorethanol und (b) einem Telomeralkohol ist, der die Formel

$$X(CF_2)_pCH_2OH$$

aufweist, in der X aus Wasserstoff und Fluor ausgewählt ist und p eine ganze Zahl von 2 bis 12 ist sowie Mischungen davon.

9. Verfahren nach Anspruch 8, bei dem X Wasserstoff ist.

10. Verfahren nach Anspruch 3, bei dem das Phosphonitrilchloridpolymer ein im wesentlichen lineares Polymer ist, das mindestens 100 (PNCl$_2$) Einheiten enthält.

11. Verfahren nach Anspruch 3, bei dem das Phosphonitrilchloridpolymer ein cyclisches Chlorpolymer ist, das etwa 3-7 (PNCl$_2$) Einheiten enthält.

12. Verfahren nach Anspruch 11, bei dem das cyclische Chlorpolymer im wesentlichen Trimer, Tetramer oder deren Mischungen ist.